# EUROPÄISCHE PATENTSCHRIFT

(11) **EP 1 201 304 B1**
(45) Veröffentlichungstag und Bekanntmachung des Hinweises auf die Patenterteilung: **16.08.2006**
(21) Anmeldenummer: 01124957.0
(22) Anmeldetag: 19.10.2001
(51) Int. Cl.: B01L 3/00, B01J 19/00

(54) **Mikrostrukturierte Plattform für die Untersuchung einer Flüssigkeit**
Microstructured platform for examining a liquid
Plateforme microstructurée pour l'examen d'un liquide

(30) Priorität: 25.10.2000 US 243246 P; 16.07.2001 US 305824 P
(43) Veröffentlichungstag der Anmeldung: 02.05.2002
(73) Patentinhaber: Boehringer Ingelheim microParts GmbH, 44227 Dortmund (DE)
(72) Erfinder: Peters, Dr. Ralf-Peter, 51467 Bergisch-Gladbach (DE); Blankenstein Dr. Gert, 44139 Dortmund (DE); Bartos, Dr. Holger, 44388 Dortmund (DE); Schön, Christian, 44229 Dortmund (DE); Osterloh,Dirk, 59423 Unna (DE); Wyzgol, Dr. Raimund, 44388 Dortmund (DE)

(56) Entgegenhaltungen:
- US-A- 5 486 335
- US-A- 5 866 345
- US-A- 6 068 752

## Beschreibung

Die Erfindung betrifft eine mit Mikrostrukturen versehene Plattform, im folgenden "Mikrochip" genannt, mit der eine Flüssigkeit untersucht werden kann, und die für weitere Anwendungen geeignet ist. Der Mikrochip ermöglicht, die Strömung der Flüssigkeit in den Hohlräumen der Mikrostruktur zu steuern. Die Flüssigkeit kann beispielsweise eine Flüssigkeit biologischer Herkunft sein.

Die Erfindung bezweckt, eine gattungsgemäße Plattform an die Erfordernisse der Untersuchung anzupassen, wobei der Aufwand für die Herstellung der Plattform in vertretbarem Rahmen zu halten ist.

Die Entwicklung der Technik zur Untersuchung von Flüssigkeiten biologischer Herkunft wird die biologische Forschung nachhaltig beeinflussen. Mit einer Anordnung immobilisierter Biomoleküle auf einer Platte lassen sich viele hunderte von Tests gleichzeitig und in miniaturisiertem Maßstab durchführen. Damit läßt sich an einer einzigen Probe eine Vielzahl von Ergebnissen schnell erhalten. In vielen Fallen können bekannte biologische Nachweisvertahren nicht eingesetzt werden, weil dafür die verfügbare Probe zu klein ist, oder weil der zeitliche oder der apparative Aufwand für die Untersuchung einer größeren Probe wesentlich zu groß ist.

Mikrochips als Substrate für analytische Untersuchungen werden die zukünftige Analysentechnik wesentlich verändern. Mit geringem Aufwand hergestellte Mikrochips werden sich auf vielen Gebieten durchsetzen, auf denen eine einfach durchzuführende und schnelle Analyse sehr kleiner Proben erforderlich ist Hierzu gehören medizinische, klinische, biochemische, chemische und industrielle Analysen sowie Analysen von Nahrungsmitteln oder im Umweltschutz. Auf vielen dieser Gebiete sind herkömmliche Untersuchungsverfahren wegen des Zeitbedarfs oder des Aufwandes nur begrenzt einsetzbar oder nicht möglich, wenn nur Proben mit geringem Volumen vorliegen.

Die bisher bekannten Mikrochips erfordern jedoch einen erheblichen Aufwand für ihre Herstellung in reproduzierbarer Form und in höher Qualität. Weiter haben die bekannten Mikrochips oft eine begrenzte Empfindlichkeit oder liefern wenig miteinander übereinstimmende Ergebnisse. Weiter ist die Zuführung der Flüssigkeit in den Mikrochip, ihre Bewegung innerhalb des Mikrochip und ihre Ableitung aus dem Mikrochip in vielen Fällen schwierig.

In US - 5 866 345 sind eine Vorrichtung und ein Verfahren zum Nachweisen eines Analyten in einer Flüssigkeit beschrieben. Dort ist das mchrstufige Verzweigen eines Kanals in Kanäle mit stufenweise abnehmender Querschnittsfläche bei stufenweise zunehmender Anzahl von Kanälen angegeben.

Damit stellt sich die Aufgabe, einen Mikrochip bereitzustellen, der für eine Vielzahl unterschiedlicher Anwendungen in miniaturisiertem Maßstab, einschließlich analytischer Untersuchungen, verwendbar ist, und bei dem - ie nach Erfordernis - die Zuführung der Flussigkeit, die Bewegung der Flüssigkeit innerhalb des Mikrochip sowie die Ableitung der Flüssigkeit aus dem Mikrochip mit hinreichender Genauigkeit gesteuert werden kann.

Diese Aufgabe wird erfindungsgemäß gelöst durch einen Mikrochip für die Untersuchung von Flüssigkeiten und andere Anwendungen, der mindestens einen mikrostrukturierten Füllbereich, mindestens einen mikrostrukturierten Untersuchungsbereich und mindestens ein mikrostrukturiertes Kanalsystem umfasst. Der Füllbereich, in dem die Kapillarkraft wirkt, enthält eine Einfüllkammer für die zu untersuchende Flüssigkeit. Im Untersuchungsbereich wird die Flüssigkeit zum Beispiel optisch untersucht. Gegebenenfalls ist ein Sammelbereich für die nicht mehr benötigte Flüssigkeitvorhanden, die aus dem Untersuchungsbereich austritt.

Der Mikrochip umfasst mindestens eine fluidische Struktur aus der Gruppe der Gabelungsstrukturen, Schmetterlingsstrukturen und Kaskadenstrukturen, mit der die Strömung der Flüssigkeit durch die mikrostrukturierten Bereiche und das Kanalsystem gesteuert wird. Der mindestens eine Untersuchungsbereich ist an mindestens einem seiner Enden über nündestens ein Kanalsystem mit dem mindestens einen Füllbereich fluidisch verbunden. Das mindestens eineKanalsystem beginnt an dem mindestens einen Füllbemich mit einem schmalen Kanal und endet an einem einzigen Untersuchungsbemich, der als breiter Kanal ausgebildet ist.

Mit der Gabelungsstrukturwird der Flüssigkeitsstrom gegabelt. Weiter kann der Mikrochip eine Verzogerungsstruktur für die voreilende Randströmung, sowie eine auf der Kapillarkraft beruhende Strukturumfassen, mit der die Flüssigkeitsströmung angehalten werden kann.. Gegebenenfalls kann ein Verdunstungsschutz für die Flüssigkeitvorgesehen sein. Weiter ist es zweckmäßig, wenn ein Auslaß für aus dem Untersuchungsbereich austretende Flüssigkeit vorhanden ist. Der Flüssigkeitssammelraum für aus dem Untersuchungsbereich austretende Flüssigkeit kann mit einer Einlaßstruktur versehen sein. Gegebenenfalls ist eine - bevorzugt hydrophobe - Entlüftungsstrukturvorhanden.

Der Füllbereich kann mindestens eine Einfüllöffnung und gegebenenfalls einen Einlaßkanal eine Füllkammer und einen Auslaßkanal umfassen. Der Einlaßkanal verbindet die Einfüllöffnung mit der Füllkammer, deren Volumen vorzugsweise hinreichend groß ist, um die gesamte Flüssigkeitsprobe (oder zumindest etwa 95 % der Flüssigkeitsprobe) aufzunehmen. Die Füllkammer ist mittels eines Auslaßkanals mit dem Untersuchungsbereich verbunden. Der Anfang des Auslaßkanals liegt bevorzugt gegenüber dem Ende des Einlaßkanals.

Der Untersuchungsbereich kann einen Untersuchungskanal umfassen, dessen Einlaß mit dem Füllbereich verbunden ist. Das Volumen des Untersuchungskanals kann bevorzugt kleiner sein als das Volumen der Einfüllöffnung oder das Volumen der Füllkammer. Die Querschnittsfläche, die Länge und die Form des Untersuchungskanals können an den Verwendungszweck des Mikrochip angepaßt sein.

Der Sammelbereich kann mindestens einen Einlaß für die Flüssigkeit, die den Untersucbungsbereich verläßt, umfassen. Der Flüssigkeitmmmelbeech kann weiter eine Sammelkammer für die Flüssigkeit enthalten sowie einen Verbindungskanal zwischen dem Ende des Untersuchungskanals und dem Sammelbereich.

Der Füllbereich ermöglicht das Füllen des Untersuchungsbereiches in vorherbestimmter Weise entweder allein unter Einwirkung der Kapillarkraft oder unter Einwirkung externer Kräfte. Die Anordnung des Füllbereiches erlaubt das vollständige Füllen des Untersuchungsbereiches ohne Einschluß von Luftblasen unabhängig von der Geschicklichkeit der Bedienungsperson.

Die Flüssigkeit kann einerseits allein durch die Kapillarkraft in den Mikrochip eingebracht werden und durch den Mikrochip strömen. Andererseits kann es zweckmäßig sein, den Mikrochip unter Einwirkung eines Druckes zu füllen oder die Flüssigkeitsströmung mittels eines Druckes zum Beispiel zum Überwinden einer auf der Kapillarkraft beruhenden Struktur zum Anhalten der Flüssigkeit zu einem vorgegebenen Zeitpunkt wieder in Gang zu setzen. Der Druck kann ein an der Einfüllöffnung wirkender Überduck sein, oder er kann ein an der Entlüftungsöffnung wirkender Unterdruck sein.

Die Einfüllkammer hat folgende Funktion und Wirkung: Das vorgegebene Volumen einer Flüssigkeit wird zum Beispiel mittels einer Pipette in die Einfüllöffnung eingegeben. Die Pipettenspitze kann dicht in die trichterförmige Einfüllöffnung gedrückt werden. Die Flüssigkeit tritt in den Verbindungskanal zwischen Einfüllöffnung und Einfüllkammer ein, wobei gegebenenfalls mittels der Pipette ein geringer Druck auf die Flüssigkeit ausgeübt werden kann Nach dem Füllen der Einfüllkammer mit Flüssigkeit kann die Pipette abgenommen werden.

Das Volumen der Einfüllkammer ist abhängig von der Auslegung des Mikrochip und seiner beabsichtigten Verwendung. Das Volumen kann in einem breiten Bereich geändert werden, zum Beispiel von etwa 1 Mikroliter bis etwa 1000 Mikroliter, bevorzugt von etwa 1 Mikroliter bis etwa 100 Mikroliter.

Der Untersuchungsbereich hat folgende Funktion und Wirkung: Der Untersuchungsbereich umfaßt im wesentlichen einen geschlossenen Kanal, dessen Länge sowie Form und Größe des Querschnitts vorgegeben sind. Für die Form des Kanals kommen unterschiedliche Ausführungen in Betracht, zum Beispiel ein gerader Hohlraum oder ein kurvenförmig angeordneter Raum. Am Ausgang des Untersuchungsbereiches können weitere fluidische Strukturen vorhanden sein. Der Hohlraum des Untersuchungsbereiches kann in Abhängigkeit vom Verhältnis der Hohlraumbreite zur Hohlraumhöhe und von Eigenschaften der Flüssigkeit entweder allein unter Einwirkung der Kapillarkraft und/oder zusätzlich durch aktiven Antrieb der Flüssigkeit gefüllt werden.

Innerhalb des Hohlraumes des Untersuchungsbereiches können beispielsweise chemische Reaktionen, Bioreaktionen, Hybridisierungsreaktionen oder andere Vorgänge ablaufen, die eine Änderung von bevorzugt optischen Eigenschaften der Flüssigkeit im Untersuchungsbereich zur Folge haben. Derartige Eigenschaften können mit bekannten optischen Verfahren erkannt werden Der Sammelbereich ist vorgesehen zum Ableiten von Flüssigkeit, die aus dem Untersuchungsbereich austritt, und vorzugsweise zum Sammeln dieser Flüssigkeit in einem Sammelraum innerhalb des Sammelbereiches.

Während des Füllens der Einfüllkammer und während des anschließenden Füllens des Untersuchungsbereiches sowie gegebenenfalls während des teilweisen Füllens des Flüssigkeitssammelraumes werden die Kanäle und Hohlräume durch die Entlüftungsöffnung entlüftet.

Der erfindungsgemäBe Mikrochip ist ein fluidisches "geschlossenes" System, bei dem sich die Flüssigkeit in einem umschlossenen Raum befindet. Der Mikrochip umfaßt eine Eimullöfmung für die Flüssigkeit und eine Entlüftungsöffnung, aus der die Luft beim Einfüllen der Flüssigkeit entweichen kann. Zwischen diesen beiden Öffnungen befindet sich ein System von Kanälen und Hohlräumen, in denen überwiegend die Kapillarkraft wirkt. Die Austrittsstelle für die Flüssigkeit ist bevorzugt mit einem Flüssigkeitssammelraum innerhalb des Mikrochip verbunden, in dem verbrauchte Flüssigkeiten gesammelt werden.

Der Füllbereich, der Untersuchungsbereich und der Sammelbereich können erfindungsgemäß - gegebenenfalls mit mehreren - fluidischen Strukturen versehen sein, die den Transport der Flüssigkeit durch die genannten Bereiche gezielt steuern.

Der Mikrochip kann im Füllbereich mit einer Einfüllkammer für die zu untersuchende Flüssigkeit versehen sein, die die Strömung der Flüssigkeit durch den Mikrochip unter Einwirkung der Kapillarkraft begünstigt. Die Einfüllkammer kann beispielsweise ein vertikal angeordneter keilförmiger Schlitz sein, dessen Breite mit zunehmender Tiefe abnimmt, oder ein trichterförmiger Einlaß, dessen Durchmesser mit zunehmender Tiefe abnimmt. Die Flüssigkeit kann mittels einer Pipette oder auf andere Weise in die Einfüllkammer gegeben werden. Der Flüssigkeitsstrom durch den Mikrochip wird durch die Kapillarkraft bewirkt.

Mit einer Schmetterlingsstruktur oder einer Kaskadenstruktur kann ein Flüssigkeitsstrom, der aus einem schmalen Kanal in einen breiten Kanal übertritt, über den Querschnitt des breiten Kanals gleichmäßig verteilt werden. Weiter kann mit diesen Strukturen ein Flüssigkeitsstrom, der aus einem breiten Kanal in einen schmalen Kanal übertritt, über den Querschnitt des breiten Kanals gleichmäßig abgezogen werden.

Die Schmetterlingsstruktur und die Kaskadenstruktur oder eine Kombination dieser Strukturen ermöglichen
- die gleichmäßige Verbreiterung des Flüssigkeitsstromes in eine mit gleichmäßiger Geschwindigkeit strömende Flüssigkeitsschicht,
- die homogene Benetzung der Oberfläche des Untersuchungsbereiches, zum Beispiel einer Hybridisierungskammer,
- die Einströmung der Flüssigkeit in den Untersuchungsbereich und/oder eine Reaktionskammer und/oder einen Nachweisbereich und/oder einen Anzeigebereich mit einem homogenen Strömungsprofil zwischen zwei Platten (dem Deckel und dem Boden des Mikrochip), und
- das gleichförmige Zusammenführen des breiten Flüssigkeitsstromes, der den Untersuchungsbereich verläßt, in einen engen Kanal.

Die Schmetterlingsstruktur besteht aus einem symmetrischen V-förmigen oder deltaförmigen Paar von Kanälen, in die sich ein einzelner schmaler Kanal verzweigt. Die beiden verzweigten Kanäle haben zusammen dieselbe Querschnittsfläche wie der einzelne schmale Kanal, der die Flüssigkeit den beiden verzweigten Kanälen zuführt. Bei Bedarf können mehrere derartige Schmetterlingsstrukturen hintereinander angeordnet sein, bevor die Flüssigkeit in den breiten Kanal eintritt.

Die Kaskadenstruktur kann aus einer dreieckförmigen Struktur bestehen, an deren Spitze der schmale Kanal angeschlossen ist An der Basis des Dreiecks ist der breite Kanal angeschlossen. Innerhalb der Kaskadenstruktur sind im allgemeinen mehrere terrassenförmige Stufen vorhanden. Bei jeder Stufe nimmt in Richtung auf die Basis des Dreiecks die Höhe der Kanalstufe ab, und die Breite der Kanalstufe nimmt zu. Dadurch ändert sich die Kapillarkraft. In der Kaskadenstruktur wird zunächst eine Stufe vollständig mit Flüssigkeit gefüllt, bevor die Flüssigkeit in die nächste Stufe eintritt. Bei einer Flüssigkeitsströmung in Richtung von der Spitze zur Basis der Kaskadenstruktur wird der Flüssigkeitsstrom von der Spitze des Dreiecks bis zur Basis gleichmäßig verbreitert und dem breiten Kanal in voller Breite zugeführt. Im breiten Kanal entsteht ein gerichteter unidirektionaler Flüssigkeitsstrom. Die Kaskadenstruktur kann auch in umgekehrter Richtung mit dem Flüssigkeitsstrom beschickt werden.

Eine Verzögerungsstruktur für die voreilende Randströmung kann die größere Strömungsgeschwindigkeit im Bereich der Ränder des Strömungskanals an die Strömungsgeschwindigkeit im mittleren Bereich des Strömungskanals angleichen. Eine voreilende Randströmung kann auftreten, wenn die Querschnittsform des Strömungskanals von der Kreisform oder der quadratischen Form stark abweicht, wenn also die Breite des Strömungskanals sehr viel größer ist als dessen Höhe. Eine Verzögerungsstruktur kann unregelmäßig geformt sein, zum Beispiel dreieckig oder sägezahnförmig. Sie ragt vom Rand des Strömungskanals in den Strömungskanal hinein. Damit läßt sich auch in einem flachen und breiten Strömungskanal eine über den ganzen Kanalquerschnitt annähernd gleich große Strömungsgeschwindigkeit erreichen.

Mit Verzögerungsstrukturen für die voreilende Randströmung können die Schwierigkeiten behoben werden, die mit der Verbreiterung der Flüssigkeitsströmung in mikrostrukturierten Kanälen verbunden sind Wenn die Flüssigkeit beispielsweise in einen breiten und flachen Kanal zwischen zwei Platten eintritt, wie zwischen den Deckel und den Boden eines Kanals, neigt die Flüssigkeit dazu, an den Rändern eines derartigen Strömungsbereiches schneller zu fließen als im mittleren Bereich, weil die Kapillarkraft in den Kanten der Ränder größer ist als im mittleren Bereich. Die voreilende Randströmung entsteht, wenn die Flüssigkeit in den Randbereichen sehr schnell davonschießt. Dadurch entsteht eine konkave Strömungsfront mit nicht homogener Strömungsgeschwindigkeit.

Bevorzugte Ausführungen des Mikrochip enthalten eine oder mehrere Verzögerungsstrukturen für die voreilende Randströmung, die eine nur wenig gekrümmte Strömungsfront und eine homogene Strömungsgeschwindigkeit bewirken. Die Verzögerungsstrukturen sind unregelmäßig geformte Strukturen, bevorzugt dreieckige oder sägezahnförmige Strukturen, die entlang der Ränder des Strömungskanals angebracht sind, und die die voreilende Randströmung an den Rändern eines breiten flachen Kanals, zum Beispiel im Untersuchungsbereich, unterdrücken und eine homogene Strömung in den Kanal hinein und durch den Kanal hindurch bewirken. Diese Strukturen stören die Wirkung der Kapillarkraft entlang der Ränder des Kanals. Es kann hinreichend sein, Verzögerungsstrukturen nur an kritischen Stellen anzubringen, zum Beispiel am Ende jedes Randes zwischen dem Ende der Schmetterlingsstruktur und dem Anfang der Hybridisierungskammer. Weiter ist es möglich, mehrere Verzögerungsstrukturen entlang der Ränder eines breiten flachen Kanals anzubringen, zum Beispiel im Untersuchungsbereich. Die Wirkung der Verzögerungsstrukturen hängt von dem Spitzenwinkel und der Höhe des "Zahnes" ab. Mit zunehmender Zahnhöhe nimmt seine Verzögerungswixktung zu.

Im Laufe einer mit dem erfindungsgemäßen Mikrochip durchgeführten Untersuchung kann es erforderlich sein, die innerhalb des Mikrochip strömende Flüssigkeit an einer vorgegebenen Stelle für eine vorgegebene Dauer anzuhalten. Eine derartige Forderung kann beispielsweise gestellt werden bei einer chemischen Reaktion oder bei einem physikalischen Vorgang wie Erwärmen oder Kühlen. Beim Erwärmen der Flüssigkeit innerhalb des Mikrochip ist deren Wärmeausdehnung zu beachten, die Kräfte erzeugen kann, die größer sind als die übliche Kapillarkraft.

Der erfindungsgemäße Mikrochip kann ferner eine auf der Kapillarkraft beruhende Struktur enthalten, mit der die Flüssigkeitsströmung an einer festgelegten Stelle angehalten werden kann. Diese Struktur umfaßt zum Beispiel einen Kanal oder einen Raum mit kleiner Kapillarkraft am Ende eines Kanals oder eines Raumes mit großer Kapillarkraft, oder er umfaßt einen Kanal oder Raum mit kleiner Kapillarkraft zwischen zwei Kanälen mit großer Kapillarkraft. Der Flüssigkeitsstrom kommt am Ende des Kanals mit großer Kapillarkraft zum Stillstand, und die Flüssigkeit tritt in den Raum mit kleiner Kapillarkraft nicht ein.

Neben einer Struktur, mit der die Flüssigkeitsströmung angehalten werden kann, kann der Mikrochip eine Struktur enthalten, mit der der Flüssigkeitsstrom verzweigt werden kann, und mit dem eine kontinuierliche Strömung durch eine Gabelungsstruktur erreicht werden kann. Die Verzweigung eine Flüssigkeitsstromes unter Benutzung einer T-förmigen Verzweigung ist normalerweise unzuverlässig wegen der unvermeidlichen Verbreiterung des Kanals, wodurch die Flüssigkeit erfahrungsgemäß angehalten wird.

Der erfindungsgemäße Mikrochip kann einen Flüssigkeitssammelraum enthalten, der zum Beispiel den verbrauchten Teil der Probenflüssigkeit oder eine Waschflüssigkeit aufnehmen kann, die aus dem Untersuchungsbereich austritt. Die Einlaßstruktur für den Flüssigkeitssammelraum kann einen Einlaß enthalten, der mit Kerben versehen ist, und der mit einem die Flüssigkeit absorbierenden Material innerhalb des Sammelraums in Berührung stehen kann, zum Beispiel mit einem Vlies.

Der Mikrochip kann ferner mit einer Entlüftungsstruktur versehen sein, die mit einem luftdurchlässigen Material abgedeckt sein kann. Ein dafür geeignetes Material ist beispielsweise eine hydrophobe Kunststoff-Membran oder ein gesintertes KunststoffPulver. Eine derartige Abdeckung ermöglicht die Entlüftung des Mikrochip beim Einffülen von Flüssigkeit und das Entgasen der eingefüllten Flüssigkeit. Die Abdeckung kann ferner zum Anhalten der Flüssigkeitsströmung beim Füllen des Mikrochip mit Flüssigkeit dienen sowie als Markierung für die vollständige Füllung des Mikrochip mit Flüssigkeit.

Bei einem Mikrochip, speziell bei miniaturisierten Untersuchungen von Flüssigkeiten, ist es erforderlich, die Flüssigkeit aus einem engen Kanal in einen weiten Kanal homogen überzuleiten. Es ist häufig erforderlich, die Flüssigkeit zwischen Strukturen mit sehr unterschiedlichen Querschnitten zu verteilen, zum Beispiel zwischen einem engen Zuleitungskanal und einem weiten Hybridisierungsbereich oder einer weiten Reaktionskammer.

Der erfindungsgemäße Mikrochip kann eine oder bevorzugt mehrere oder alle oben genannten fluidischen Strukturen enthalten, also eine Schmetterlingsstruktur, eine Kaskadenstruktur, eine Gabelungsstruktur für die Gabelung des Flüssigkeitsstroms, eine Verzögerungsstruktur für die voreilende Randströmung, eine auf der Kapillarkraft beruhende Struktur zum Anhalten der Flüssigkeitsströmung, gegebenenfalls mit Verdunstungsschutz für die Flüssigkeit, einen Auslaß für aus dem Untersuchungsbereich austretende Flüssigkeit, eine Einlaßstruktur für den Flüssigkeitssammelraum, einen im Mikrochip vorhandenen Sammelraum für aus dem Untersuchungsbereich austretende Flüssigkeit, eine hydrophobe Entlüftungsstruktur und weitere der aufgeführten Merkmale.

Der erfindungsgemäße Mikrochip ist geeignet für Anwendungen oder Untersuchungen von in den Untersuchungsbereich eingebrachten Flüssigkeiten, die Biomoleküle enthalten, wie Nukleinsäuren, Peptide und ähnliche Moleküle.

Die Erfindung wird an Hand der Figuren weiter erläutert.

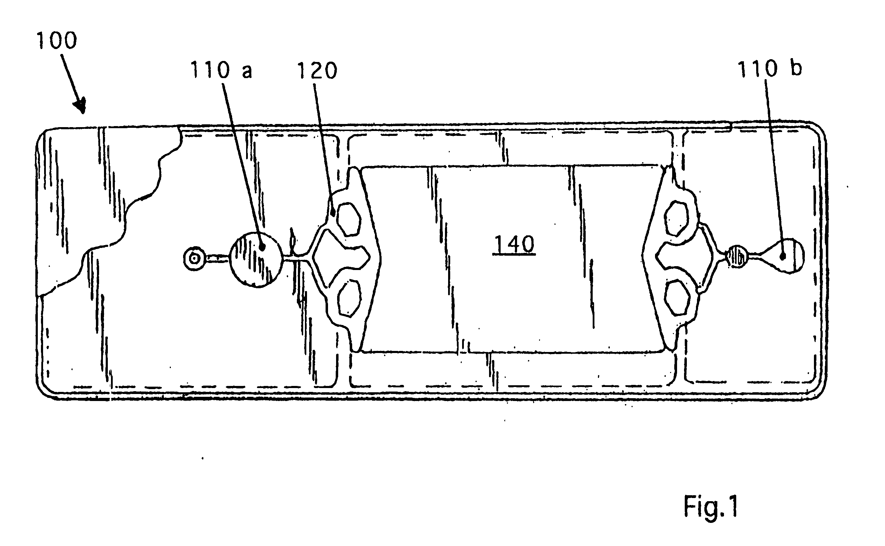

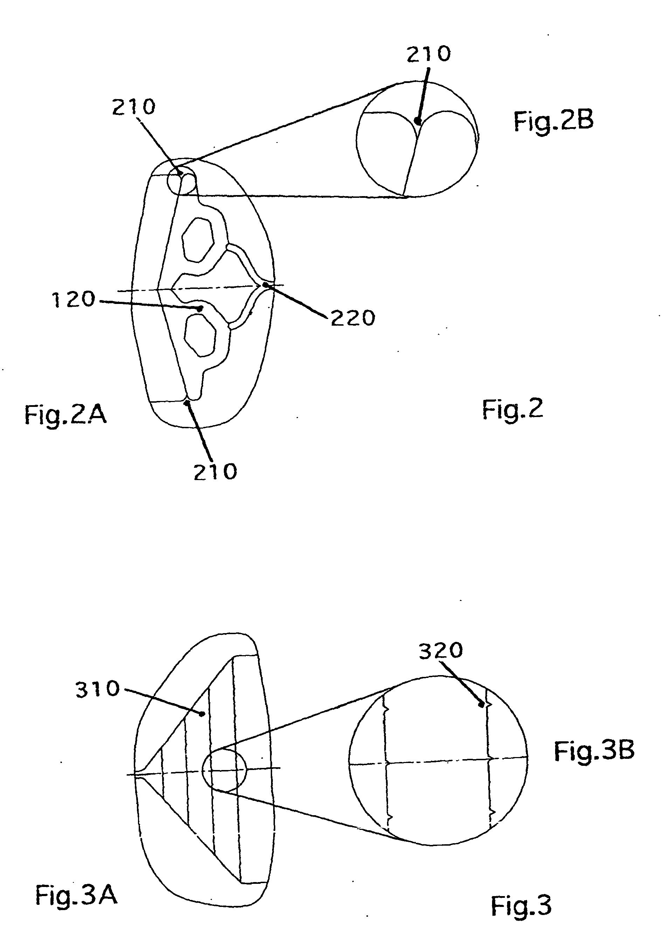

Fig.1 zeigt einen typischen Mikrochip für analytische Zwecke von oben gesehen. Der Mikrochip 100 enthält eine Öffnung 110 a zum Einfüllen einer Probenflüssigkeit und eine Öffnung 110 b zum Spülen des Kanals 140 und der Verbindungskanäle mit einer Pufferflüssigkeit und zum Entlüften während des Einfüllens. Die Öffnungen können sehr unterschiedlich angeordnet sein und können das Einfüllen einer Flüssigkeit ohne Beeinträchtigung des Untersuchungsbereiches ermöglichen. Die Einfüllöffnungen können trichterförmig sein, wobei das weite Ende des Trichters nach außen und das enge Ende nach innen gekehrt ist, und wodurch das Einfüllen der Flüssigkeit in den Mikrochip erleichtert wird Die Struktur 120 wird in Figur 2 beschrieben.

Fig. 2 (mit den Teilfiguren 2A und 2B) zeigt eine typische Schmetterlingsstruktur 120, eine Verzögerungsstruktur für die voreilende Randströmung 210 und eine Gabelungsstruktur 220.

Der Vorteil der Gabelungsstruktur 220 liegt im wesentlichen in der Form der Gabelung, nämlich der Y-förmigen Verzweigung des Kanalsystems. Im Gegensatz zu einer ungeeigneten T-förmigen Verzweigung ist erfindungsgemäß eine gebogene V-förmige Verzweigung vorgesehen, bei der "die Spitze des V" tief in den Zuleinmgskanal hineinreicht. Die "Spitze des V" kann eine dreieckförmige scharfe Kante innerhalb der Verzweigung sein. Weil die "Spitze des V" in den Zuleitungskanal tief hineinragt (wodurch die Y-Struktur entsteht), wird die Kapillarkraft nicht unterbrochen wie in der üblichen T-förmigen Verzweigung, und der Flüssigkeitsstrom bleibt erhalten.

Fig. 3 (mit den Teilfiguren 3A und 3B) zeigt eine typische Kaskadenstruktur mit Terrassen 310 mit unterschiedlicher Höhe und Kerben 320.

Die Schwierigkeiten beim Überleiten der Flüssigkeit von einer Mikrostruktur in eine anschließende Mikrostruktur mit erheblich unterschiedlichem Querschnitt können mittels der in den Figuren 2 und 3 angegebenen fluidischen Strukturen behoben werden. Die Schmetterlingsstruktur ist ein symmetrisches delta-förmiges System 120 von gegabelten Kanälen mit konstanter Querschnittsfläche. Mit zunehmender Anzahl der Gabelungen nimmt die Kanaltiefe ab und die Kanalbreite nimmt zu. Die Schmetterlingsstruktur beginnt oder endet mit einer V-förmigen Kante am breiten Ende der baumartigen Struktur.

Eine konstante Querschnittsfläche kann sowohl eine konstante Strömungsgeschwindigkeit als auch eine erhöhte Kapillarkraft bewirken. Die V-förmige Kante am breiten Ende der Baumstruktur kann den Effekt der konkaven Form der Strömungsfront beseitigen; bei diesem Effekt eilt die Flüssigkeit in den Randbereichen des breiten Strömungskanals der Flüssigkeit im mittleren Bereich des Strömungskanals voraus. Eine ungleichförmige Kanaltiefe ermöglicht die Verteilung der Flüssigkeit zu einer homogen strömenden Flüssigkeitsschicht, wodurch die homogene Strömung der Flüssigkeit in den Untersuchungsbereich erreicht und die konkave Form der Strömungsfront abgeschwächt wird. Die V-förmige Kante am breiten Ende der Baumstruktur begradigt die Strömungsfront. Die Spitze in der Mitte der V-förmigen Kante kann scharf oder gerundet sein.

Die Schmetterlingsstruktur kann weiter zum gleichförmigen Überleiten der Flüssigkeit aus einem breiten Kanal in einen engen Kanal benutzt werden.

Bevorzugte Ausführungen des erfindungsgemäßen Mikrochip können eine dreieckförmige Kaskadenstruktur mit Stufen (Terrassen) 310 enthalten, bei der die Stufenhöhe in Richtung zur Spitze des Dreiecks zunimmt. Die in dieser Richtung abnehmende Kapillarkraft ermöglicht - je nach Strömungsrichtung der Flüssigkeit durch die Kaskadenstruktur - die homogene Verbreiterung oder Verengung des Flüssigkeitsstromes. Die Kaskade hat zumindest zwei Bereiche mit unterschiedlicher Tiefe und unterschiedlicher Kapillarkraft. Die Flüssigkeit füllt jede Stufe zunächst vollständig aus, bevor sie in die folgende Stufe übertritt. Die Stufenkanten der Kaskadenstruktur können mit Kerben versehen sein wie sie für die Einlaßstruktur zum Sammelraum beschrieben sind. Die Kerben erleichtern die Benetzung der folgenden Stufe.

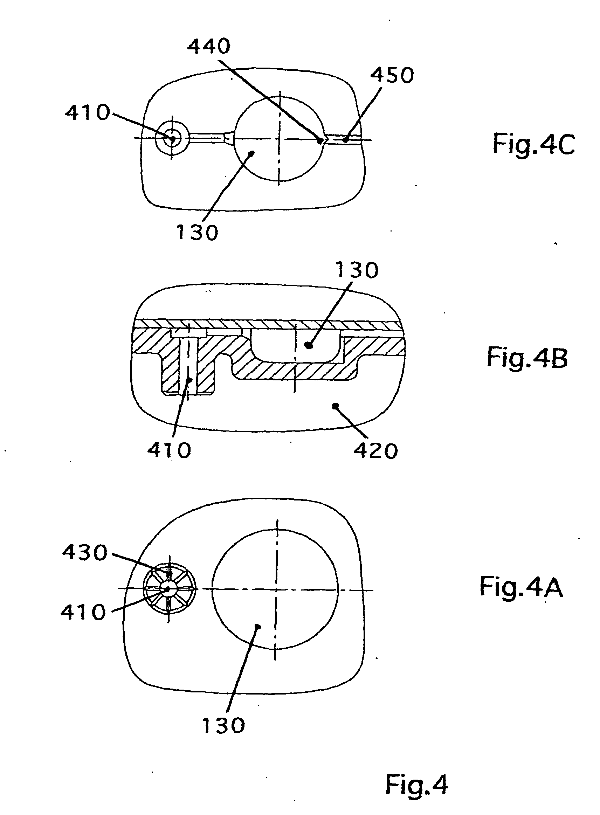

Fig. 4 zeigt eine Einlaßstruktur für den Flüssigkeitssammelraum, und zwar von unten gesehen (Fig. 4A), als Längsschnitt (Fig. 4B) und von oben gesehen (Fig. 4C). Um die Einlaßstruktur mit Flüssigkeit zu füllen, ist eine Kammer 130 zweckmäßig, in der die Kapillarkraft wirkt. Die Kammer ist mit einer vertikal angeordneten keilförmigen Kapillarkerbe 440 versehen, die sich vom Boden der Kammer bis zum Eintritt des Zuleitungskanals 450 in die Kammer erstreckt. Die Kammer 130 wird mit Flüssigkeit gefüllt, wobei die Kapillarkraft der vertikalen Kerbe wirkt. Die Kapillarkraft einer derartigen Kerbe verändert sich mit dem Winkel an der Spitze der Kerbe.

Der Mikrochip kann gegebenenfalls einen integrierten Flüssigkeitssammelraum 420 und eine Einlaßstruktur 410 enthalten, wobei die Innenwände der Einlaßstruktur mit Kerben versehen sind, die ein absorbierendes Material, wie Gewebe, Vlies, oder saugfähiges Material, festhalten, das die nicht mehr benötigte Flüssigkeit aufsaugt und deren Rückströmung in den Untersuchungsbereich verhindert.

Zwecks wirksamer Ankopplung des fluidischen Systems an das absorbierende Material im Sammelbereich ist der Hals der Einlaßstruktur bereichsweise mit Kerben 430 versehen, die bevorzugt sternförmig angeordnet sind. Diese Kerben können als Koppelstruktur dienen, die die Kontaktfläche zwischen der Einlaßöffnung und dem absorbierenden Material vergrößern. Die keilförmigen Kerben erzeugen eine anfängliche Saugkraft auf die anstehende Flüssigkeit wegen der erhöhten Kapillarkraft.

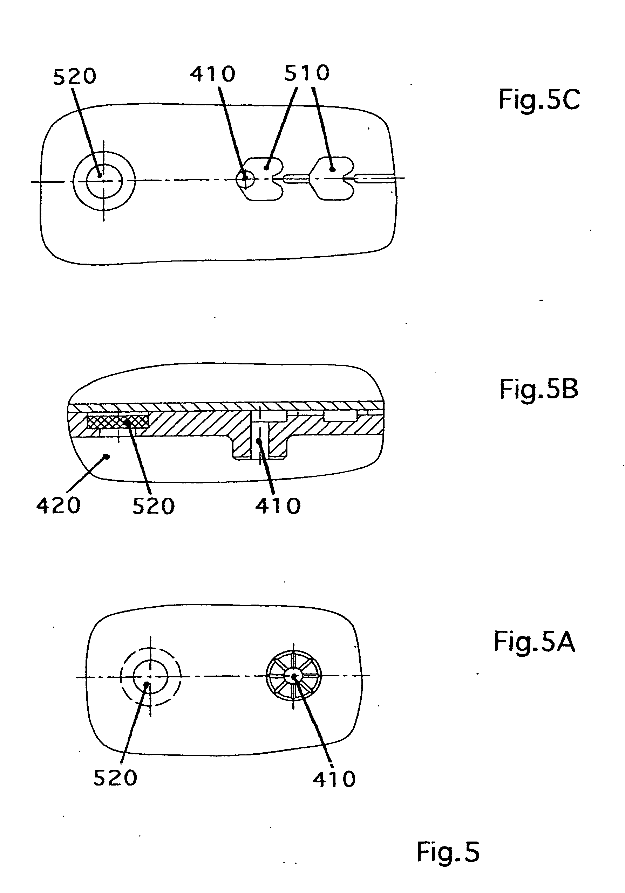

Fig. 5 zeigt eine vor dem Einlaß 410 in den Flüssigkeitssammelraum angeordnete typische Struktur 510, mit der die Flüssigkeitsströmung angehalten werden kann, sowie eine hydrophobe Batlurrungsstruktur 520, und zwar von unten gesehen (Fig. 5A), als Längsschnitt (Fig. 5B) und von oben gesehen (Fig. 5C). Bevorzugt wird eine Struktur verwendet, die aus einer Kombination von zwei Räumen 510 mit niedriger Kapillarkraft besteht, vor denen jeweils ein Kanal mit großer Kapillarkraft angeordnet ist. Eine solche Struktur, die vor einer Einlaßstruktur für den Sammelraum angeordnet ist, ist in Fig. 5B und 5C dargestellt. Der erste Kanal - in Strömungsrichtung der Flüssigkeit gesehen - hält die während des Füllvorganges in den Mikrochip einströmende Flüssigkeit an. Der zweite Kanal mit großer Xapillarkraft hält die Flüssigkeit zum Beispiel während eines im Untenuchungsverfahren erforderlichen Aufheizvorganges an. Mit einer derartigen Anordnung kann die Flüssigkeit jeweils vor und nach einer thermischen Ausdehnung angehalten werden.

Die Auslaßöffnung 520 ist bevorzugt mit einem hydrophoben luftdurchlässigen Material abgedeckt, welches das Entweichen von Luft ermöglicht und das Austreten von Flüssigkeit verhindert.

Weiter Strukturen zum Anhalten der Flüssigkeitsströmung können an anderen Stellen des Mikrochip vorgesehen werden, zum Beispiel in Fig. 1 zwischen den Öffnungen 110a und 110b. Eine an dieser Stelle vorgesehene Struktur verbindert die Rückströmung von Flüssigkeit aus dem Bereich um die Öffnung 110a in Richtung zur Öffnung 110b.

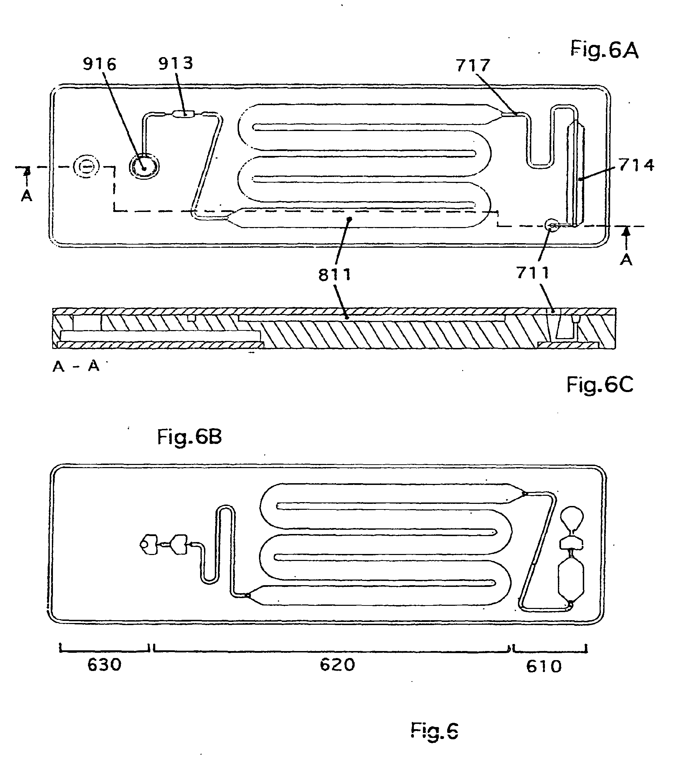

Fig 6 zeigt zwei unterschiedlich strukturierte Mikrochips von oben gesehen (Fig. 6A und 6B) mit einem Füllbereich 610, einem Untersuchungsbereich 620 und einem Sammelbereich 630 für Flüssigkeit, die aus dem Untersuchungsbereich austritt. Fig. 6C ist eine Ansicht im Längsschnitt durch den Mikrochip gemäß Fig. 6A entlang der Linie A - A . Die Ausführung gemäß Fig. 6A hat eine Einlaßöffnung 711 am Eingang zur Füllkammer 714, eine Kapillare 717, die den Ausgang der Füllkammer 714 mit dem Eingang zum Kanal 811 im Untersuchungsbereich verbindet, einen breiten Kanal 913 zwischen zwei Mikrokanälen und eine mit Kerben versehene Einlaßstruktur 916 für den Sammelraum.

Fig. 6B zeigt einen Mikrochip, bei dem die fluidischen Strukturen in anderer Weise als in Fig. 6A angeordnet sind.

Fig 6C zeigt einen Längsschnitt durch den Mikrochip gemäß Fig. 6A entlang der Linie A-A.

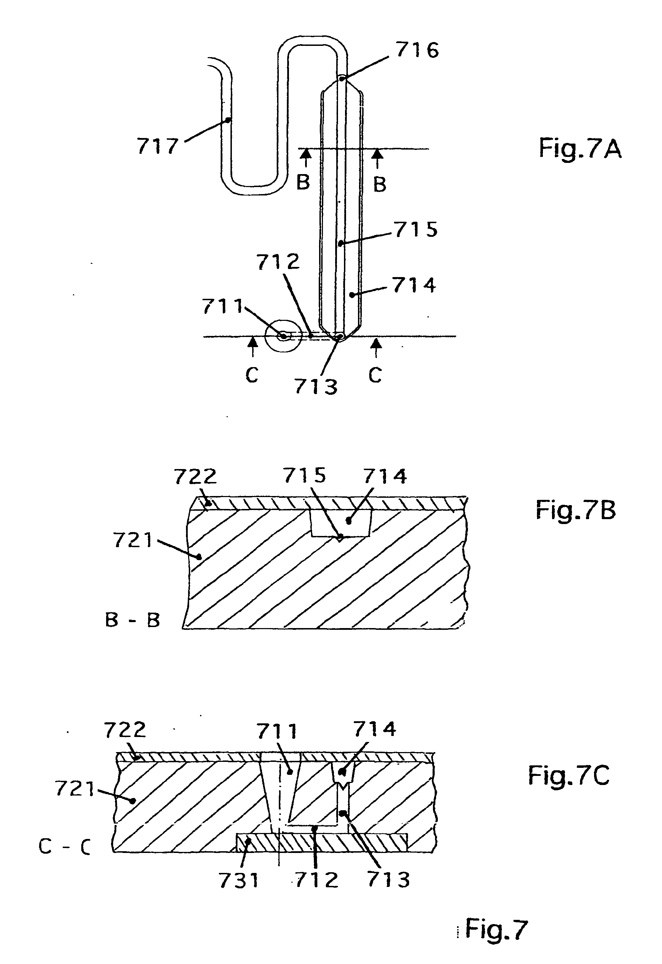

Fig. 7 zeigt Einzelheiten eines typischen Einfüllbereichs. Fig. 7A zeigt den Einfüllbereich von oben gesehen. Fig 7B und Fig. 7C zeigen Querschnitte durch den Füllbereich entlang der Linie B - B beziehungsweise C - C in Fig. 7A. Die trichterförmige Einlaßöffnung 711 kann die Spitze einer Pipette aufnehmen. Die Einlaßöffnung ist über den Bodenkanal 712 und einen vertikalen Kanal 713 mit dem einen Ende der Füllkammer 714 verbunden. Am Boden der Fülikammer ist ein V-förmiger Graben 715 vorgesehen, der sich über die gesamte Länge der Füllkammer erstreckt. An der Übergangsstelle von der Füllkammer in die Verbindungskapillare 717 befindet sich eine Kapillarstufe 716.

Die Einlaßöffnung 711 kann die gesamte einzufüllende Menge an Flüssigkeit aufnehmen und diese vorübergehend zurückhalten. Von dieser Einlaßöffnung ausgehend wird zunächst die Füllkammer 714 unter Einwirkung der Kapillarkraft gleichmäßig mit Flüssigkeit gefüllt sowie anschließend die Verbindungskapillare 717 und die daran angeschlossenen Hohlräume.

Die in Fig. 7A dargestellten Strukturen sind auf einer Plattform 721 angeordnet. Die Oberseite dieser Plattform ist mit der Deckplatte 722 bedeckt, die die Oberseite der Plattform und alle auf der Oberseite angeordneten Mikrostrukturen abdeckt mit Ausnahme der Einlaßöffnung und der Entlüftungsöffnung. Der Bodenkanal 712 ist mit der Deckplatte 731 auf der Unterseite der Plattform 721 bedeckt.

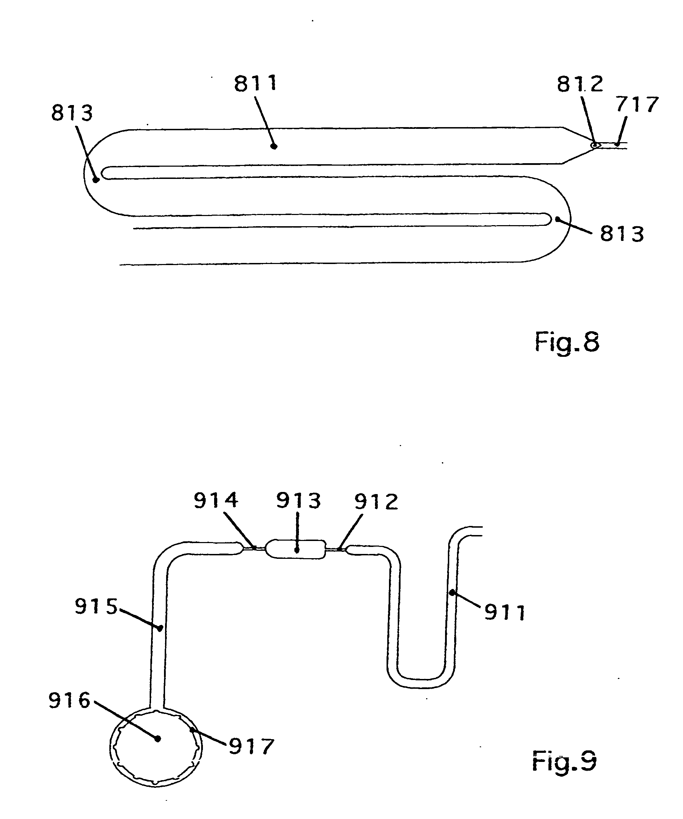

Fig 8 zeigt Einzelheiten im Untersuchungsbereich von oben gesehen. Der nur teilweise dargestellte Kanal 811 im Untersuchungsbereich ist mittels der Kapillare 717 mit dem Füllbereich verbunden. An der Übergangsstelle der Kapillare 717 in den Kanal 811 befindet sich eine Kapillarsteufe 812. Die Breite des Kanals 811 in den Kurven 813 ist kleiner als die Breite im geraden Teil des Kanals 811. Dadurch wird die anderenfalls im Bereich der Kurven inhomogene Geschwindigkeit der Flüssigkeitsströmung vergleichmäßigt.

Fig. 9 zeigt eine weitere bevorzugte Ausführungsform einer auf der Kapillarkraft beruhenden Struktur, mit der die Flüssigkeitsströmung angehalten wird. Diese Struktur ist in Fig. 9 stromabwärts des Untersuchungsbereiches angeordnet. Die vergleichsweise weite Kapillare 911 geht in eine kurze Mikrokapillare 912 mit einer engen Öffnung über, auf die die weite Kammer 913, eine weitere kurze Mikrokapillare 914 und eine vergleichsweise weite Verbindungskapillare 915 folgen. Die Kapillare 915 dient als Auslaßkanal für die aus dem Untersuchungsbereich austretende Flüssigkeit. Die Kapillare 915 kann mit der Einlaßstruktur 916 für den (nicht dargestellten) Sammelraum verbunden sein. Die Einlaßstruktur zum Sammelraum kann mit Kerben 917 versehen sein. Die Binlaßstruktur und der hohle Sammelraum können integrale Bestandteile der Plattform 721 sein.

Die Strukturen 912, 913 und 914 können zum Steuern der Flüssigkeitsströmung dienen, mit denen die Strömung angehalten und wieder in Gang gesetzt werden kann. Diese Strukturen können weiter als Diffusionsbarrieren zwischen der Verbindungskapillare 911 und der Verbindungskapillare 915 dienen wegen der herabgesetzten Querschnittsfläche der Mikrokapillaren 912 und 914 und des vergleichsweise großen Volumens der dazwischen liegenden weiten Kammer 913.

Die Strukturen 912, 913 und 914 können andererseits am Ende des Füllbereiches angeordnet sein. In diesem Fall entspricht die Verbinduagskapillare 915 in Fig. 9 der Verbindungskapillare 717 in Fig. 8, und die Kapillare 911 in Fig. 9 ist mit dem einen Ende des Füllkanals 714 in Fig. 7A verbunden. Dadurch wird der Untersuchungsbereich vom Fünbereich fluidisch besser getrennt, und das "Übersprechen" der im Füllbereich vorhandenen Flüssigkeit mit der im Untersuchungsbereich vorhandenen Flüssigkeit wird heaabgesetzt.

Die weite Kammer 913 kann zum Entgasen der Flüssigkeit dienen. Dieses ist besonders wichtig, wenn zum Beispiel ein übliches Verfahren zum Hybridisieren von Nukleinsäuren und anderen Substanzen durchgeführt wird, bei dem die Temperatur innerhalb eines großen Bereiches variiert wird. Ein Beispiel aus der üblichen Molekularbiologie ist die Verwendung eines thermozyklischen Verfahrens zum Replizieren von Nukleinsäuren wie die Polymerase-Kettenreaktion (PCR), bei dem die Temperatur von 25 °C bis 90 °C variiert wird.

Typische Abmessungen des erfindungsgemäßen Mikrochip, wie er in den Fig. 1 und 6 dargestellt ist, sind: Breite etwa 25 Millimeter, Länge etwa 75 Millimeter und Dicke etwa 2 Millimeter. Typische Abmessungen des Kanals 811 in Fig 6 sind: Breite etwa 3 Millimeter und Höhe etwa 50 Mikrometer. Typische Abmessungen des Kanals 140 in Fig. 1 sind: Breite etwa 20 Millimeter, Höhe etwa 75 Mikometer.

Der Mikrochip enthält eine oder mehrere der oben genannten Mikrostrukturen, mit denen die Flüssigkeitsströmung im Mikrochip gesteuert werden kann. Dazu gehören eine Schmetterlingsstruktur 120, eine Kaskadenstruktur 310, eine Gabelungsstruktur 220, eine Verzögerungsstruktur 210, eine Einlaßstruktur 130 und 410 für den Flüssigkeitssammelraum, eine Struktur 510, mit der die FlüssigkeitsStrömung angehalten werden kann, und eine hydrophobe Entlüftungsstruktur 520.

Der erfindungsgemäße Mikrochip kann aus einer Vielzahl unterschiedlicher Materialien hergestellt werden, zum Beispiel aus Glas, Quarz, Polymeren, einem Gel, Kunststoffen, Harzen, Kohlenstoff, Metall, oder aus einer Kombination verschiedener Materialien, wie Polymermischungen, mit einem Polymer beschichteten Glas, mit Siliziumoxid beschichteten Metall.

Der Mikrochip kann in unterschiedlichen Formen und Größen hergestellt werden, die die leichte Handhabung des Mikrochip und seine Verwendbarkeit mit einer Vielzahl bekannter Laborgeräte ermöglichen, wie Mikrotiterplatten, Pipettier- und Dosierautomaten, Mikroskopen, array spotters, Geräte zur photolithographischen Herstellung, Flachbett-Abtaster oder -Leser, Fluoreszenzdetektoren, Infrarotdetektoren, Massenspektrometer, thermocyclers, Hybridisierungsöfen, auf Laborautomaten ablaufende Verfahren. Der Mikrochip kann fast jede geeignete Form haben, wie beispielsweise ein Quadrat, Rechteck, Kreis, Kugel, Scheibe, Zylinder, Objektträger, Folie, Platte, Rohr. Ein im wesentlichen flacher Mikrochip ist vorzugsweise mit hervorstehenden oder eingedrückten Bereichen oder Vertiefungen versehen, die seine Handhabung erleichtern.

Der erfindungsgemäße Mikrochip kann nach bekannten Verfahren hergestellt werden, beispielsweise durch Laserbearbeitung, Heißprägen, mechanische Bearbeitung oder Ätzen. Ein Mikrochip aus Kunststoff wird bevorzugt durch Spritzgießen hergestellt.

Der erfindungsgemäße Mikrochip ist als geschlossene Anordnung von Mikrostrukturen ausgelegt. Die zu untersuchende Flüssigkeit ist in einem im wesentlichen flüssigkeitsdichten Behälter enthalten, der mit integrierten mikrofluidischen Strukturen zum Einfüllen einer Flüssigkeitsprobe und zum Waschen des Behälters versehen ist.

Die Strömung einer Flüssigkeit durch die Hohlräume des Mikrochip kann allein durch die Kapillarkraft bewirkt werden. Die Flüssigkeitsströmung kann ferner durch einen von außen einwirkenden Druck erzeugt werden, oder durch andere Kräfte, wie die Zentrifugalkraft, Schwerkraft, elektrische Kraft, osmotische Kraft, elektroosmotische Kraft. Solche Antriebskräfte können für sich allein genommen oder in verschiedenen Kombinationen miteinander angewendet werden.

## Patentansprüche

1. Mikrochip für die Untersuchung von Flüssigkeiten und andere Anwendungen, der mindestens einen mikrostrukturierten Füllbereich mindestens einen mikrostrukturierten Untersuchungsbereich und mindestens ein mikrostrukturiertes Kanalsystem umfasst, wobei
• der Mikrochip mindestens eine fluidische Struktur aus der Gruppe der Gabelungsstrukturen. Schmetterlingsstrukturen und Kaskadenstrukturen umfasst, mit der die Strömung der Flüssigkeit durch die mikrostrukturierten Bereiche und das Kanalsystem gesteuert wird, und
• der mindestens eine Untersuchunsbereich an mindestens einem seiner Enden über mindestens ein Kanalsystem mit dem mindestens einen Füllbereich fluidisch verbunden ist und
• das mindestens eine Kanalsystem an dem mindestens einen Füllbereich mit einem schmalen Kanal beginnt und an einem einzigen Untersuchungsbereich endet der als breiter Kanal ausgebildet ist.

2. Mikrochip nach Anspruch 1, wobei
• die fluidischen Strukturen weiter mindestens eine Verzögerungsstruktur für die voreilende Randströmung umfassen.

3. Mikrochip nach Anspruch 1, wobei
• die fluidischen Strukturen weiter mindestens eine auf der Kapillarkraft beruhende Struktur umfassen.

4. Mikrochip nach Anspruch 1, wobei
• ein einzelner Strömungskanal in zwei Strömungskanäle gegabelt ist und die beiden gegabelten Kanäle mit dem einzelnen Kanal Y-förmig angeordnet sind und die Gabelung bevorzugt eine gebogenen V-förmige Struktur hat und die Spitze des V sich in den einen Kanal erstreckt, wodurch in der Gabelung eine kontinuierliche Kapillarkraft erhalten bleibt und die gegabelten Kanäle ein symmetrisches Delta bilden, und die Querschnittsfläche des einzelnen Kanals im wesentlichen genau so groß ist wie die Querschnittsfläche der beiden gegabelten Kanäle zusammengenommen.

5. Mikrochip nach Anspruch 3, wobei
• das Kanalsystem mit einer V-förmigen Kante am breiten Ende des Delta abschließt.

6. Mikrochip nach Anspruch 1, wobei
• ein Kanal eine dreieckförmige Struktur mit zwei oder mehr Stufen oder Terrassen hat, deren Tiefe mit zunehmender Breite der dreieckförmigen Struktur abnimmt, wodurch die Kapillarkraft ansteigt und die homogene Verbreiterung eines Flüssigkeitsstromes oder die homogene Zusammenführung zweier Flüssigkeitsströme erreicht wird.

7. Mikrochip nach Anspruch 5, wobei
• die Kanten der Stufen oder Terrassen mit kerbförmigen Strukturen versehen sind.

8. Mikrochip nach Anspruch 1, wobei
• die Ränder eines Kanals mit einer oder mehreren Verzögenmgsstrukturen für die voreilende Randströmung versehen sind, die die homogene Strömung ermöglichen, und die unregelmäßig geformt sind, bevorzugt dreieckförmige oder sägezahnförmige Strukturen.

9. Mikrochip nach Anspruch 7, wobei
• die Verzögenmgsstrukturen einen Winkel von 1 Grad bis etwa 120 Grad haben.

10. Mikrochip nach Anspruch 7, wobei
• die Verzögerungsstrukturen eine Höhe von etwa 1 Mikrometer bis etwa 3 Millimeter haben.

11. Mikrochip nach Anspruch 1, wobei
• der Füllbereich eine Einfüllkammer mit mindestens einer vertikalen keilförmigen Kapillarkerbe enthält und sich die Kapillarkerbe vom Boden der Einfüllkammer bis zum Eingang eines Verbindungskanals erstreckt, und der Verbindungskanal mittels der Kapillarkraft aus der Füllkammer mit Flüssigkeit gefüllt wird.

12. Mikrochip nach Anspruch 11, wobei
• die Kapillarkerbe einen Winkel von etwa 1 Grad bis etwa 150 Grad hat.

13. Mikrochip nach Anspruch 1, wobei
• der Mikrochip eine oder mehrere Kapillaren enthält, die zwischen Kanälen oder Räumen angeordnet sind, in den eine kleine Kapillarlaaft wirkt, wodurch die Flüssigkeitsströmung am Ende einer Kapillare angehalten wird.

14. Mikrochip nach Anspruch 1, wobei
• die Kanäle mehrere Gabelungen nacheinander enthalten, bei denen ein Kanal in zwei Kanäle gegabelt ist, und mit zunehmender Anzahl der Gabelungen die Kanaltiefe abnimmt und die Kanalbreite zunimmt.

15. Mikrochip nach Anspruch 1, wobei
• der Mikrochip weiter einen Sammelbereich für die aus dem Untersuchungsbereich austretende Flüssigkeit enthält, und der Sammelbereich integraler Bestandteil des Mikrochip ist.

16. Mikrochip nach Anspruch 15, wobei
• der Sammelbereich einen Sammelraum enthält, dessen Einlaß mit einer oder mehreren kerbförmigen Strukturen versehen ist.

17. Mikrochip nach Anspruch 16, wobei
• ein absorbierendes Material, bevorzugt ein Vlies, innerhalb der mit Kerben versehenen Zonen des Sammelbereiches vorgesehen ist.

18. Mikrochip nach Anspruch 1, wobei
• der Mikrochip eine Entlüftungsöffnung enthält, die bevorzugt mit einem luftdurchlässigen flüssigkeitsdichten Material bedeckt ist

19. Mikrochip nach Anspruch 18, wobei
• die Entlüftungsöffnung an einer Stelle des Mikrochip angebracht ist, an der Gasblasen aus der im Mikrochip enthaltenen Flüssigkeit entweichen können.

## Revendications

1. Micropuce pour l'examen de fluides et autres applications, qui comprend au moins une région de remplissage micro-structurée, au moins une région d'examen micro-structurée et au moins un système de canaux micro-structuré, dans laquelle
• la micropuce comprend au moins une structure fluidique choisie dans le groupe des structures de ramification, des structures en papillon et des structures en cascade, qui permettent l'écoulement du fluide par les régions micro-structurées et le système de canaux,
• la région d'examen est, de ses extremités, reliée de manière fluidique à la région de remplissage par l'intermédiaire d'au moins un système de canaux, et
• le système de canaux commence par un canal étroit au niveau de la région de remplissage et se termine dans une seule région d'examen formée par un canal large.

2. Micropuce selon la revendication 1, dans laquelle
• les structures fluidiques comprennent en outre au moins une structure de ralentissement pour l'écoulement prématuré en bordure.

3. Micropuce selon la revendication 1, dans laquelle
• les structures fluidiques comprennent en outre au moins une structure reposant sur la force capillaire.

4. Micropuce selon la revendication 1, dans laquelle
• un canal unique d'écoulement est ramifié en deux canaux d'écoulement et les deux canaux ramifiés sont disposés en forme de Y avec le seul canal, la ramification ayant de préférence une structure courbée en forme de V et la pointe du V s'étendant dans le canal unique ce qui permet de maintenir une force capillaire continue dans la ramification et permet aux canaux ramifiés de former un delta symétrique, la surface en coupe transversale du canal unique étant pour l'essentiel aussi grande que la surface en coupe transversale des deux canaux ramifiés ensemble.

5. Micropuce selon la revendication 3, dans laquelle
• le système de canaux se termine par une arête en forme de V à l'extrémité large du delta.

6. Micropuce selon la revendication 1, dans laquelle
• un canal a une structure triangulaire avec deux ou plusieurs étages ou terrasses, dont la profondeur décroit lorsque la largeur de la structure triangulaire croit, ce qui permet d'augmenter la force capillaire et d'atteindre un élargissement homogène du courant de fluide ou un guidage homogène conjoint de deux courants de fluide.

7. Micropuce selon la revendication 5, dans laquelle
• les arêtes des étages ou des terrasses sont munies de structures en forme d'encoche.

8. Micropuce selon la revendication 1, dans laquelle
• pour l'écoulement prématuré en bordure, les bords d'un canal sont munis d'une ou de plusieurs structures de ralentissement permettant un écoulement homogène et qui sont formées de manière irrégulière, de préférence des structures triangulaires ou en dents de scie.

9. Micropuce selon la revendication 7, dans laquelle
• les structures de ralentissement ont un angle de 1 degré à environ 120 degrés.

10. Micropuce selon la revendication 7, dans laquelle
• les structures de ralentissement ont une hauteur de 1 micromètre environ à 3 millimètres environ.

11. Micropuce selon la revendication 1, dans laquelle
• la région de remplissage contient une chambre de remplissage avec au moins une encoche capillaire verticale angulaire et l'encoche capillaire s'étend depuis le fond de la chambre de remplissage jusqu'à l'entrée d'un canal de liaison, et le canal de liaison est rempli de fluide au moyen de la force capillaire provenant de la chambre de remplissage.

12. Micropuce selon la revendication 11, dans laquelle
• l'encoche capillaire a un angle de 1 degré environ a 150 degrés environ.

13. Micropuce selon la revendication 1, dans laquelle
• la micropuce contient un ou plusieurs capillaires disposés entre des canaux ou des espaces dans lesquels agit une faible force capillaire qui permet d'immobiliser l'écoulement de fluide à l'extrémité d'un capillaire.

14. Micropuce selon la revendication 1, dans laquelle
• les canaux contiennent plusieurs ramifications les unes derrière les autres, dont un canal est ramifié en deux canaux et, lorsque le nombre de ramifications croît, la profondeur des canaux diminue mais la largeur des canaux augmente.

15. Micropuce selon la revendication 1, dans laquelle
• la micropuce contlent en outre une région collectrice pour le fluide sortant de la région d'examen et la région collectrice est un élément formé d'une seule pièce avec la micropuce.

16. Micropuce selon la revendication 15, dans laquelle
• la région collectrice contient un espace collecteur dont l'admission est munie d'une ou de plusieurs structures en forme d'encoche.

17. Micropuce selon la revendication 16, dans laquelle
• un matériau absorbant, de préférence un non-tissé, est prévu à l'intérieur des zones de la région collectrice qui sont munies d'encoches.

18. Micropuce selon la revendication 1, dans laquelle
• la micropuce contient une ouverture d'aération recouverte de préférence d'un matériau poreux étanche.

19. Micropuce selon la revendication 18, dans laquelle
• l'ouverture d'aération est appliquée à un endroit de la micropuce d'où peuvent s'échapper des bulles gazeuses provenant du fluide contenu dans la micropuce.

## Claims

1. Microchip for the examination of liquids and other applications, which comprises at least one microstructured filling region, at least one microstructured examination region and at least one microstructured channel system, in which
• the microchip comprising at least one fluidic structure selected from the group consisting of forked structures, butterfly structures and cascaded structures, by which the flow of the liquid through the microstructured regions and the channel system is controlled, and
• the at least one examination region, at at least one of its ends, being fluid-connected via at least one dhannel system to the at least one filling region, and
• the at least one channel system beginning at the at least one filling region with a narrow channel and ending at a single examination region which is in the form of a wide channel.

2. Microchip according to claim 1, in which
• the fluidic structures also comprise at least one deceleration structure for the leading boundary flow.

3. Microchip according to claim 1, in which
• the fluidic structures also comprise at least one structure based on the capillary force.

4. Microchip according to claim 1, in which
• a single flow channel is forked into two flow channels, and the two forked channels together with the single channel are arranged in a Y-shape, and the fork preferably has a curved Vshaped structure, with the tip of the V extending into the one channel, with the result that a continuous capillary force is maintained in the fork, and the forked channels form a symmetric delta, and the cross-sectional area of the single channel is substantially precisely the same as the cross-sectional area of the two forked channels together.

5. Microchip according to claim 3, in which
• the channel system ends with a V-shaped edge at the wide end of the delta.

6. Microchip according to claim 1, in which
• a channel has a triangular structure with two or more steps or terraces, the depth of which decreases with increasing width of the triangular structure, with the result that the capillary force increases and the homogenous widening of a liquid stream or the homogenous combining of two liquid streams is achieved.

7. Microchip according to claim 5, in which
• the edges of the steps or terraces are provided with notch-like structures.

8. Microchip according to claim 1, in which
• the edges of a channel are provided with one or more deceleration structures for the leading boundary flow, which allow homogenous flow and are irregular in form, preferably triangular or sawtooth structures.

9. Microchip according1 to claim 7, in which
• the deceleration structures form an angle of from 1 degree to approximately 120 degrees.

10. Microchip according to claim 7, in which
• the deceleration structures have a height of approximately 1 micrometre to approximately 3 millimetres.

11. Microchip according to claim 1, in which
• the filling region includes a fill-up chamber with at least one vertical wedge-shape capillary notch, and the capillary notch extends from the bottom of the fill-up chamber to the entry of a connecting channel, and the connecting channel is filled with liquid from the filling' chamber by means of the capillary force.

12. Microchip according to claim 11, in which
• the capillary notch forms an angle of approximately 1 degree to approximately 150 degrees.

13. Microchip according to claim 1, in which
• the microchip includes one or more capillaries which are arranged between channels or spaces in which a low capillary force is active, with the result that the liquid flow is stopped at the end of a capillary.

14. Microchip according to claim 1, in which
• the channels include a plurality of forks in succession, at which forks a channel is forked into two channels, and' as the number of forks increases, the channel depth decreases and the channel width increases.

15. Microchip according to claim 1, in which
• the microchip also includes a collection region for the liquid emerging from the examination region, and the collection region is an integral part of the microchip.

16. Microchip according to claim 15, in which
• the collection region includes a collection space, the inlet of which is provided with one or more notch-like structures.

17. Microchip according to claim 16, in which
• an absorbent material, preferably a nonwoven, is provided within those zones of the collection region which are provided with notches.

18. Microchip accordant? to claim 1, in which
• the microchip includes a vent opening, which is preferably covered with an air-permeable, liquid-tight material.

19. Microchip according to claim 18, in which
• the vent opening is arranged at a position on the microchip at which gas bubbles can escape from the liquid contained in the microchip.
